# EUROPÄISCHE PATENTANMELDUNG

(11) **EP 2 186 531 A2**
(43) Veröffentlichungstag der Anmeldung: **19.05.2010**
(21) Anmeldenummer: 09174332.8
(22) Anmeldetag: 28.10.2009
(51) Int. Cl.: A61L 31/16, A61L 31/10

(54) **Erhöhung der Effizienz pharmazeutische Wirkstoffe-freisetzender Medizinprodukte durch Kombination mit einem Inhibitor des Transportproteins P-Glycoprotein**

(30) Priorität: 13.11.2008 DE 102008043724
(71) Anmelder: Biotronik VI Patent AG, 6341 Baar (CH)
(72) Erfinder: Gratz, Matthias, 91054, Erlangen (DE); Borck, Alexander, 91086, Aurachtal (DE)
(74) Vertreter: Lindner-Vogt, Karin L.

(57) **Zusammenfassung**

Die vorliegende Erfindung betrifft pharmazeutische Wirkstoffe-freisetzende Medizinprodukte, deren Effizienz durch Kombination mit einem Inhibitor des Transportproteins P-Glycoprotein erhöht wird.

## Beschreibung

Die vorliegende Erfindung betrifft pharmazeutische Wirkstoffe-freisetzende Medizinprodukte, deren Effizienz durch Kombination mit einem Inhibitor des Transportproteins P-Glycoprotein erhöht wird.

Eine der häufigsten Todesursachen in der entwickelten Welt sind kardiovaskuläre Erkrankungen, wobei Koronarerkrankungen von höchster Bedeutung sind. Zur Behandlung dieser Erkrankungen werden intravaskulär Gefäßprothesen, wie zum Beispiel Ballons oder Stents, in das betroffene Blutgefäß eines Patienten eingebracht und gegebenenfalls implantiert, um dieses aufzuweiten und offen zu halten.

Allerdings kann es aufgrund des intravaskulären Eingriffs zu einer erhöhten Thrombusbildung sowie einer erhöhten Proliferation von glatten Muskelzellen kommen, was zu einem erneuten Gefäßverschluss (Restenose) führen kann. Überschießende Proliferation von Narbengewebe führt bei ca. 30 - 40% aller unbeschichteten Stents nach längerer Zeit zu einer Restenose.

Um die Risikofaktoren einer Restenose zu verhindern, wurde eine Vielzahl an Beschichtungen für Stents entwickelt, die eine erhöhte Hämokompatibilität bieten sollen. Seit längerer Zeit werden bspw. in die Beschichtung der Stents antikoagulierende, antimikrobielle, antiinflammatorische und antiproliferative Agenzien einzeln oder in Kombination eingesetzt. Diese Substanzen sollen aus dem Beschichtungsmaterial des Stents derart freigesetzt werden, dass sie Entzündungen des umliegenden Gewebes, überschießendes Wachstum der glatten Muskelzellen oder das Verklumpen von Blut verhindern.

Allerdings haben viele dieser beschichteten Stents den Nachteil, dass die jeweiligen Wirkstoffe aufgrund von Resistenzerscheinungen, die auf körpereigene Strukturen zurückzuführen sind, in einer erhöhten Konzentration eingesetzt werden müssen, was zu einer lokalen Intoxikation führen kann.

Diese Resistenzerscheinungen, auch Multidrug-Resistance (MDR) genannt, werden von verschiedenen membranständigen Transportproteinen hervorgerufen, die unter Aufwendung von Energie dazu befähigt werden, Stoffe direkt aus der Membran zu entfernen. Das wesentliche Charakteristikum der Transport-basierten Resistenzmechanismen besteht in einer Verringerung der intrazellulären Wirkstoffkonzentration.

Einer der bedeutendsten Faktoren der Multidrug-Resistance ist das Transportprotein P-Glycoprotein. Das P-Glycoprotein ist aus dem Grund von besonderer Bedeutung, da es in der Lage ist, eine Vielzahl von Verbindungen, die den unterschiedlichsten Strukturklassen angehören, zu erkennen und aus dem intrazellulären Raum zu transportieren.

Seit der Entdeckung dieser für die MDR verantwortlichen Transportproteine sind erhebliche Anstrengungen unternommen worden, Wirkstoffe auf ihre P-Glycoprotein-selektiven inhibitorischen Eigenschaften zu untersuchen und in beschichtete medizinische Vorrichtungen einzubringen. Durch die Hemmung von P-Glycoproteinen kann auf diese Weise die Akkumulation des intrazellulären Wirkstoffs erhöht und damit die Multidrug-Resistance verringert werden.

So werden unter anderem in der DE 600 28 747 T, DE 10 2004 020 856 A, DE 600 26 513 T und DE 601 21 992 T diverse medizinische Vorrichtungen offenbart, unter anderem Stents, die mit einer Polymerbeschichtung enthaltend eine Kombination von verschiedenen Arzneimittel, unter anderem Inhibitoren des Transportproteins P-Glycoprotein, versehen werden können. Die Polymerbeschichtung kann aus einer Anzahl verschiedener Polymere zusammengesetzt sein.

Es besteht allerdings nach wie vor das Problem, dass viele der eingesetzten Inhibitoren des Transportproteins P-Glycoprotein eine zu geringe Affinität gegenüber dem P-Glycoprotein aufweisen, so dass nach wie vor für eine hinreichende intrazelluläre Wirkstoffkonzentration die Wirkstoffe in erhöhter Konzentration in die jeweiligen Beschichtungen eingebracht werden müssen. Diese erhöhten Konzentrationen der Wirkstoffe können möglicherweise zu unerwünschten Nebenreaktionen auf das umliegende Zell- und Gewebematerial führen.

Aufgabe der vorliegenden Erfindung ist es somit, Medizinprodukte bereitzustellen, die durch Kombination eines hochaffinen Inhibitors des Transportproteins P-Glycoprotein mit anderen pharmazeutischen Wirkstoffen die Effizienz der Wirkstoffakkumulation im intrazellulären Raum erhöhen und den Einsatz geringerer Konzentrationen der jeweiligen Komponenten zur Vermeidung einer Intoxikation des Zell- und Gewebematerials ermöglichen.

Diese Aufgabe wird erfindungsgemäß dadurch gelöst, dass ein ganz oder in Teilen mit einer biostabilen und/oder bioabbaubaren Polymerschicht überzogenes Medizinprodukt bereitgestellt wird, welches in und/oder auf der biostabilen und/oder bioabbaubaren Polymerschicht mindestens einen Inhibitor des Transportproteins P-Glycoprotein sowie mindestens einen weiteren pharmazeutischen Wirkstoff aufweist, wobei der mindestens eine Inhibitor des Transportproteins P-Glycoprotein ausgewählt ist aus der Gruppe bestehend aus Valspodar (PSC833), Elacridar (GF120918), Tariquidar (XR9576), Zosuquidar (LY335979) und ONT-093 (OC144-093).

Im Folgenden steht der Begriff Inhibitor als Äquivalent für Inhibitor des Transportproteins P-Glycoprotein.

Es hat sich überraschenderweise gezeigt, dass durch das Ein- und/oder Aufbringen von mindestens einem Inhibitor des Transportproteins P-Glycoprotein ausgewählt aus der Gruppe bestehend aus PSC833, GF120918, XR9576, LY335979 und OC144-093 in und/oder auf der biostabilen und/oder bioabbaubaren Polymerschicht einerseits die Konzentration des Inhibitors und andererseits auch die Konzentration des mindestens einen pharmazeutischen Wirkstoffs verringert werden kann. Aufgrund der hohen Affinität von PSC833, GF120918, XR9576, LY335979 und OC144-093 gegenüber dem P-Glycoprotein, wird das P-Glycoprotein auch bei geringeren Konzentrationen des Inhibitors stärker in seiner Wirkung gehemmt, so dass ein verringerter zellauswärts-gerichteter Transport der Wirkstoffe erfolgt. Somit kann die Effizienz der erfindungsgemäßen pharmazeutische Wirkstoffe freisetzenden Medizinprodukte deutlich gesteigert werden.

Medizinprodukte innerhalb des Schutzumfangs der vorliegenden Erfindungen beinhalten beliebige medizinische Vorrichtungen, welche benutzt werden, wenigstens teilweise, um in den Körper eines Patienten eingebracht zu werden. Beispiele beinhalten implantierbare Vorrichtungen Herzschrittmacher, Katheter, Nadelinjektionskatheter, Blutgerinnselfilter, vaskuläre Transplantate, Ballons, Stenttransplantate, Gallenstents, Darmstents, Bronchiallungenstents, Speiseröhrenstents, Harnleiterstents, Aneurysmen-ausfüllende Spulen und andere Spulenvorrichtungen, transmyokardiale Revaskularisationsvorrichtungen, perkutane myokardiale Revaskularisationsvorrichtungen. Weiterhin können beliebige natürliche und/oder künstliche Medizinprodukte eingesetzt werden, beispielsweise Prothesen, Organe, Gefäße, Aorten, Herzklappen, Schläuche, Organersatzteile, Implantate, Fasern, Hohlfasern, Membranen, Konserven, Blutbehältern, Titerplatten, Adsorbermedien, Dialysatoren, Verbindungsstücke, Sensoren, Ventile, Endoskope, Filter, Pumpenkammern sowie andere Medizinprodukte, welche hämokompatible Eigenschaften aufweisen sollen. Der Begriff Medizinprodukte ist weit zu fassen und bezeichnet insbesondere solche Produkte, die kurzzeitig (z.B. Endoskope) oder dauerhaft (z.B. Stents) mit Blut in Kontakt kommen.

Besonders bevorzugte Medizinprodukte sind Ballonkatheter und Stents. Stents herkömmlicher Bauart weisen eine filigrane Stützstruktur aus metallischen Streben auf, die zur Einbringung in den Körper zunächst in einem nicht-expandierten Zustand vorliegen und die am Ort der Applikation dann in einen expandierten Zustand aufgeweitet werden. Der Stent kann vor oder nach dem Crimpen auf einen Ballon beschichtet werden.

Der Grundkörper des Stents besteht vorzugsweise aus einem metallischen Material aus einem oder mehreren Metallen aus der Gruppe Eisen, Magnesium, Nickel, Wolfram, Titan, Zirkonium, Niob, Tantal, Zink oder Silizium und ggf. einer zweiten Komponente aus einem oder mehreren Metallen aus der Gruppe Lithium, Natrium, Kalium, Kalzium, Mangan, Eisen oder Wolfram, vorzugsweise aus einer Zink-Kalziumlegierung.

In einem weiteren Ausführungsbeispiel besteht der Grundkörper aus einem Formgedächtnis-Material aus einem oder mehreren Materialien aus der Gruppe bestehend aus Nickel-Titan-Legierungen und Kupfer-Zink-Aluminium-Legierungen, vorzugsweise aber aus Nitinol.

In einem weiteren bevorzugten Ausführungsbeispiel besteht der Grundkörper des Stents aus Edelstahl, vorzugsweise aus einem Cr-Ni-Fe-Stahl - hier bevorzugt die Legierung 316L - oder einem Co-Cr-Stahl. Ferner kann der Grundkörper des Stents mindestens teilweise aus Kunststoff und/oder einer Keramik bestehen.

In einem weiteren Ausführungsbeispiel besteht der Grundkörper des Stents aus einem biokorrodierbaren metallischen Werkstoff, zum Beispiel einer biokorrodierbaren Legierung, ausgewählt aus der Gruppe Magnesium, Eisen und Wolfram; insbesondere ist der biokorrodierbare metallische Werkstoff eine Magnesiumlegierung.

Unter einer biokorrodierbaren Magnesiumlegierung wird ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung Yttrium und weitere Seltenerdmetalle, da sich eine derartige Legierung aufgrund ihrer physikochemischen Eigenschaften und hohen Biokompatibilität, insbesondere auch seiner Abbauprodukte, auszeichnet. Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

In einem weiteren Ausführungsbeispiel besteht der Stent aus natürlichen Polymeren, wie zum Beispiel aus Collagen, Chitin, Chitosan, Heparin.

Die Oberfläche des erfindungsgemäßen Medizinprodukts weist ganz oder in Teilen eine biostabile und/oder bioabbaubare Polymerschicht auf, die mindestens einen Inhibitor des Transportproteins P-Glycoprotein ausgewählt aus der Gruppe bestehend aus PSC833, GF120918, XR9576, LY335979 und OC144-093 sowie mindestens einen weiteren pharmazeutischen Wirkstoff enthält.

Als Synonym für die biostabile und/oder bioabbaubare Polymerschicht wird im Rahmen der vorliegenden Erfindung der Begriff Beschichtung oder polymere Trägermatrix verwendet.

Eine biostabile und/oder bioabbaubare Polymerschicht im Sinne der Erfindung ist eine zumindest abschnittsweise Auftragung der Komponenten der Beschichtung auf das Medizinprodukt. Vorzugsweise wird die gesamte Oberfläche des Medizinprodukts von der Beschichtung bedeckt. Die Schichtdicke liegt vorzugsweise im Bereich von 2 µm bis 60 µm, besonders bevorzugt von 10 µm bis 30 µm. Die erfindungsgemäßen Medizinprodukte zeichnen sich unter anderem dadurch aus, dass sich durch die Reduktion des Wirkstoffs, bei gleich bleibender Wirksamkeit, wesentlich dünnere Schichtdicken auf dem Medizinprodukt realisieren lassen. Im Vergleich dazu liegen Wirkstoff-beladene Beschichtungen herkömmlicher Medizinprodukte in der Größenordnung von 100 µm

Der Gewichtsanteil einer erfindungsgemäßen polymeren Trägermatrix an den die Beschichtung bildenden Komponenten der Beschichtung beträgt vorzugsweise mindestens 40%, besonders bevorzugt mindestens 70%. Der Gewichtsanteil des mindestens einen Inhibitors des Transportproteins P-Glycoprotein an den die Beschichtung bildenden Komponenten der Beschichtung beträgt bevorzugt nicht mehr als 30%, besonders bevorzugt nicht mehr als 15%. Der Gewichtsanteil des mindestens einen weiteren pharmazeutischen Wirkstoffs an den die Beschichtung bildenden Komponenten der Beschichtung beträgt bevorzugt nicht mehr als 30%, besonders bevorzugt nicht mehr als 15%.

Die Beschichtung kann direkt auf das Medizinprodukt aufgetragen werden. Die Verarbeitung kann nach Standardverfahren für die Beschichtung erfolgen. Es können einschichtige, aber auch mehrschichtige Systeme (zum Beispiel sogenannte base coat-, drug coat- oder top coat-Schichten) erstellt werden. Die Beschichtung kann unmittelbar auf dem Grundkörper des Implantats aufgebracht sein oder es sind weitere Schichten dazwischen vorgesehen, die beispielsweise der Haftvermittlung dienen.

Alternativ kann die biostabile und/oder bioabbaubare Polymerschicht, enthaltend mindestens einen Inhibitor des Transportproteins P-Glycoprotein ausgewählt aus der Gruppe bestehend aus PSC833, GF120918, XR9576, LY335979 und OC144-093 sowie mindestens einen weiteren pharmazeutischen Wirkstoff, als Kavitätenfüllung vorliegen oder Bestandteil einer Kavitätenfüllung sein. Das Medizinprodukt, insbesondere der Stent, weist zu diesem Zweck eine oder mehrere Kavitäten auf. Kavitäten befinden sich beispielsweise an der Oberfläche des Medizinprodukts und lassen sich beispielsweise durch Laserablation in Nano- bis Mikrometerdimensionen erstellen. Bei Medizinprodukten, insbesondere Stents, mit einem biodegradierbaren Grundkörper kann eine Kavität auch im Innern des Grundkörpers angeordnet sein, so dass die Freisetzung des Material erst nach Freilegung erfolgt. Der Fachmann kann sich bei der Ausgestaltung der Kavität an dem im Stand der Technik beschriebenen Systemen orientieren. Der Begriff "Kavität" umfasst dabei z.B. Löcher und Vertiefungen.

Die biostabile und/oder bioabbaubare Polymerschicht im Rahmen der vorliegenden Erfindung ist zusammengesetzt aus Polymeren ausgewählt aus der Gruppe bestehend aus nichtresorbierbaren, permanenten Polymeren und/oder resorbierbaren bioabbaubaren Polymeren.

Besonders bevorzugt ist die biostabile und/oder bioabbaubare Polymerschicht zusammengesetzt aus Polymeren ausgewählt aus der Gruppe Polyolefine, Polyetherketone, Polyether, Polyvinylalkohole, Polyvinylhalogenide, Polyvinylester, Polyacrylate, Polyhalogenolefine, Polyamide, Polyamidimide, Polysulfone, Polyester, Polyurethane, Silikone, Polyphosphazene, Polyphenylen, Polymerschäume (aus Styrolen und Carbonaten), Polydioxanone, Polyglycolide, Polylactide, Poly-ε-caprolacton, Ethylvinylacetat, Polyethylenoxid, Polyphosphorylcholin, Polyhydroxybuttersäuren, Lipide, Polysaccharide, Proteine, Polypeptide sowie Copolymere, Blends und Derivate dieser Verbindungen.

Ganz besonders bevorzugt ist die biostabile und/oder bioabbaubare Polymerschicht zusammengesetzt aus Polymeren ausgewählt aus der Gruppe bestehend aus Polypropylen, Polyethylen, Polyisobutylen, Polybutylen, Polyetheretherketon, Polyethylenglycol, Polypropylenglycol, Polyvinylalkohole, Polyvinylchlorid, Polyvinylfluorid, Polyvinylacetat, Polyethylacrylat, Polymethylacrylat, Polytetrafluorethylen, Polychlortrifluorethylen, PA 11, PA 12, PA 46, PA 66, Polyamidimide, Polyethersulfon, Polyphenylsulfon, Polycarbonate, Polybutylenterephthalat, Polyethylenterephthalat, Elastane, Pellethane, Silikone, Polyphosphazene, Polyphenylen, Polymerschäume (aus Styrolen und Carbonaten), Polydioxanone, Polyglycolide, Poly-L-, Poly-D-, und Poly-D,L-Lactid, sowie Poly-ε-caprolacton, Ethylvinylacetat, Polyethylenoxid, Polyphosphorylcholin, Polyhydroxyvalerat, Cholesterin, Cholesterinester, Alginat, Chitosan, Levan, Hyaluronsäure, Uronide, Heparin, Dextran, Cellulose, Fibrin, Albumin, Polypeptide und Copolymere, Blends und Derivate dieser Verbindungen.

Die biostabile und/oder bioabbaubare Polymerschicht richtet sich bevorzugt nach der gewünschten Elutionsgeschwindigkeit sowie den individuellen Charakteristika der verschiedenen eingesetzten Wirkstoffe und nach der unterschiedlichen Resorptions- bzw. Degradationsgeschwindigkeit am Wirkort des Medizinproduktes.

Unter einem Inhibitor im Rahmen der vorliegenden Erfindung ist ein Hemmstoff, also eine Substanz, zu verstehen, der eine oder mehrere Reaktionen - chemischer, biologischer oder physiologischer Natur - so beeinflusst, dass diese verlangsamt, gehemmt oder verhindert werden.

Der mindestens eine Inhibitor des Transportproteins P-Glycoprotein ausgewählt aus der Gruppe bestehend aus PSC833, GF120918, XR9576, LY335979 und OC144-093 wird bevorzugt in einer Konzentration zwischen 0,25 bis 7 µg/mm² Stentoberfläche, besonders bevorzugt zwischen 0,6 bis 3,8 µg/mm² Stentoberfläche in und/oder auf die biostabile und/oder bioabbaubare Polymerschicht ein- oder aufgebracht.

Der mindestens eine pharmazeutische Wirkstoff ist eine Substanz, die an die Umgebung des Implantats abgegeben werden muss, in welcher das Medizinprodukt, insbesondere ein Ballonkatheter oder ein Stent, eingeführt wird, aber es wird bevorzugt, dass sie mit sehr niedrigen Raten in den Blutkreislauf freigesetzt wird. Der pharmazeutische Wirkstoff ist bevorzugt aus den folgenden Arzneistoffklassen ausgewählt: antimikrobielle, antimitotische, antimyotische, antineoplastische, antiphlogistische, antiproliferative, antithrombotische und vasodilatatorische Mittel.

Besonders bevorzugte pharmazeutische Wirkstoffe sind Triclosan, Cephalosporin, Aminoglycosid, Nitrofurantoin, Penicilline wie Dicloxacillin, Oxacillin sowie Sulfonamide, Metronidazol, 5-Fluoruracil, Cisplatin, Vinblastin, Vincristin, Epothilone, Endostatin, Verapamil, Statine wie Cerivastatin, Atorvastatin, Simvastatin, Fluvastatin, Rosuvastatin sowie Lovastatin, Angiostatin, Angiopeptin, Taxane wie Paclitaxel, Immunsuppressiva oder - modulatoren wie z.B. Rapamycin oder dessen Derivate wie Biolimus, Everolimus, Deforloimus, Novolimus" Methotrexat, Colchicin, Flavopiridol, Suramin, Cyclosporin A, Clotrimazol, Flucytosin, Griseofulvin, Ketoconazol, Miconazol, Nystatin, Terbinafin, Steroiden wie Dexamethason, Prednisolon, Corticosteron, Budesonid, Östrogen, Hydrocortison sowie Mesalamin, Sulfasalazin, Heparin und seinen Derivaten, Urokinase, PPack, Argatroban, Aspirin, Abciximab, synthetisches Antithrombin, Bivalirudin, Enoxoparin, Hirudin, r-Hirudin, Protamin, Prourokinase, Streptokinase, Warfarin, Flavonoiden wie 7,3',4'-trimethoxyflavon sowie Dipyramidol, Trapidil sowie Nitroprusside.

Die pharmazeutischen Wirkstoffe werden einzeln oder kombiniert in gleicher oder unterschiedlicher Konzentration eingesetzt.

Besonders bevorzugt ist eine Kombination von mehreren antiproliferativen Wirkstoffen. Insbesondere bevorzugt weist das ganz oder in Teilen mit einer biostabilen und/oder bioabbaubaren Polymerschicht überzogene Medizinprodukt in und/oder auf der biostabilen und/oder bioabbaubaren Polymerschicht mindestens einen der Inhibitoren des Transportproteins P-Glycoprotein ausgewählt aus der Gruppe bestehend aus PSC833, GF120918, XR9576, LY335979 und OC144-093 sowie mindestens einen der pharmazeutischen Wirkstoffe Paclitaxel und Rapamycin, einzeln oder in Kombination, auf.

Zudem bevorzugt sind Kombinationen von antiproliferativ wirkenden Substanzen mit vasodilatatorischen oder pleiotropen Wirkstoffen. Zu derartigen Wirkstoffen zählen Verapamil sowie Statine. Insbesondere bevorzugt weist das mit einer biostabilen und/oder bioabbaubaren Polymerschicht überzogene Medizinprodukt in und/oder auf der biostabilen und/oder bioabbaubaren Polymerschicht mindestens einen der Inhibitoren des Transportproteins P-Glycoprotein ausgewählt aus der Gruppe bestehend aus PSC833, GF120918, XR9576, LY335979, OC144-093, mindestens einen der pharmazeutischen Wirkstoffe aus der Gruppe Paclitaxel und Rapamycin sowie einen weiteren pharmazeutischen Wirkstoff ausgewählt aus der Gruppe bestehend aus Verapamil, Atorvastatin, Simvastatin und Lovastatin, einzeln oder in Kombination, auf.

Der pharmazeutische Wirkstoff ist bevorzugt in einer pharmazeutisch aktiven Konzentration von 0,2 bis 3,5 µg/mm² Stentoberfläche, weiter bevorzugt von 0,25 bis 0,75 µg/mm² Stentoberfläche enthalten.

Das erfindungsgemäße Medizinprodukt kann eine weitere, innere oder äußere Beschichtung aufweisen. Eine weitere äußere Schicht kann die Beschichtung oder Kavitätenfüllung enthaltend mindestens einen Inhibitor des Transportproteins P-Glycoprotein sowie mindestens einen weiteren pharmazeutischen Wirkstoff ganz oder in Teilen bedecken. Diese äußere Beschichtung kann ein degradierendes Polymer enthalten oder daraus bestehen, insbesondere ein Polymer aus der Klasse der PLGA (poly(lactic-co-glycolic acid)) oder der PLGA-PEG Blockcopolymere. Gegebenenfalls kann in diese weitere, äußere Schicht ein weiterer Wirkstoff eingebettet sein, der frei eluieren kann oder bei Degradation der äußeren Beschichtung freigesetzt wird.

Verfahren zur Herstellung eines pharmazeutische Wirkstoffe-freisetzenden Medizinproduktes sind dem Fachmann bekannt. Beispielsweise kann ein Verfahren folgende Schritte umfassen:
a) Bereitstellen eines Medizinprodukts, insbesondere Stent oder Ballonkatheter;
b) Aufbringen einer biostabilen und/oder bioabbaubaren Polymerschicht;
   und
c) Auf- und/oder Einbringen mindestens eines Inhibitors des Transportproteins P-Glycoprotein sowie mindestens eines pharmazeutischen Wirkstoffs auf und/oder in die biostabile und/oder bioabbaubare Polymerschicht.

Ein weiterer Aspekt der Erfindung ist die Verwendung von PSC833, GF120918, XR9576, LY335979 und OC144-093 als Inhibitoren des Transportproteins P-Glycoprotein zur Herstellung eines ganz oder in Teilen mit einer biostabilen und/oder bioabbaubaren Polymerschicht überzogen Medizinproduktes, insbesondere Ballonkatheter oder Stents, wobei sich in und/oder auf der biostabilen und/oder bioabbaubaren Polymerschicht mindestens ein Inhibitor des Transportproteins P-Glycoprotein ausgewählt aus der Gruppe bestehend aus PSC833, GF120918, XR9576, LY335979 und OC144-093 sowie mindestens ein pharmazeutischer Wirkstoff befindet.

Nachfolgend wird die Erfindung anhand von Ausführungsbeispielen näher erläutert, welche den Erfindungsgegenstand aber nicht einschränken sollen.

### Ausführungsbeispiel 1: Beschichtung eines Stents mit äquimolaren Mengen an PSC833 und Paclitaxel (Ptx)

15,8 mg (0,013 mmol) PSC833 und 11 mg (0,013 mmol) Ptx werden in 100 ml Chloroform zusammen mit 100 mg Poly-L-Lactid (PLLA; L210 von Boehringer Ingelheim) bei Raumtemperatur gelöst. Als Lösungsvermittler werden dem Lösungsmittel 5 % Methanol zugesetzt. Die so hergestellte Lösung wird mit einem Tauchverfahren auf den Stent aufgebracht. Die Flächenbeladung von Ptx beträgt 0,5 µg/mm². Die Wirkstoffmenge, bestehend aus Ptx und PSC833, bezogen auf das Polymer (PLLA) beträgt 21 %.

### Ausführungsbeispiel 2: Beschichtung eines Stents mit 1.2 Äquivalent PSC833 und 1 Äquivalent Paclitaxel (Ptx)

18,95 mg (0,0156 mmol) PSC833 und 11 mg (0,013 mmol) Ptx werden in 100 ml Chloroform zusammen mit 100 mg Poly-L-Lactid (PLLA; L210 von Boehringer Ingelheim) bei Raumtemperatur gelöst. Als Lösungsvermittler werden dem Lösungsmittel 5 % Methanol zugesetzt. Die so hergestellte Lösung wird mit einem Tauchverfahren auf den Stent aufgebracht. Die Flächenbeladung von Ptx beträgt 0,3 µg/mm₂. Die Wirkstoffmenge, bestehend aus Ptx und PSC833, bezogen auf das Polymer (PLLA) beträgt 23 %.

### Ausführungsbeispiel 3: Beschichtung eines Stents mit äquimolaren Mengen an GF120918 und Paclitaxel (Ptx)

7,32 mg (0,013 mmol) GF120918 und 11 mg (0,013 mmol) Ptx werden in 100 ml Chloroform zusammen mit 100 mg Poly-L-Lactid (PLLA; L210 von Boehringer Ingelheim) bei Raumtemperatur gelöst. Als Lösungsvermittler werden dem Lösungsmittel 5 % Methanol zugesetzt. Die so hergestellte Lösung wird mit einem Tauchverfahren auf den Stent aufgebracht. Die Flächenbeladung von Ptx beträgt 0,5 µg/mm². Die Wirkstoffmenge, bestehend aus Ptx und GF120918, bezogen auf das Polymer (PLLA) beträgt 15 %.

### Ausführungsbeispiel 4: Beschichtung eines Stents mit 1.2 Äquivalent GF120918 und 1 Äquivalent Paclitaxel (Ptx)

8,79 mg (0,0156 mmol) GF120918und 11 mg (0,013 mmol) Ptx werden in 100 ml Chloroform zusammen mit 100 mg Poly-L-Lactid (PLLA; L210 von Boehringer Ingelheim) bei Raumtemperatur gelöst. Als Lösungsvermittler werden dem Lösungsmittel 5 % Methanol zugesetzt. Die so hergestellte Lösung wird mit einem Tauchverfahren auf den Stent aufgebracht. Die Flächenbeladung von Ptx beträgt 0,3 µg/mm². Die Wirkstoffmenge, bestehend aus Ptx und GF120918, bezogen auf das Polymer (PLLA) beträgt 16,5 %.

## Patentansprüche

1. Medizinprodukt, wobei die Oberfläche des Medizinprodukts ganz oder in Teilen mit einer biostabilen und/oder bioabbaubaren Polymerschicht überzogen ist und sich in und/oder auf der biostabilen und/oder bioabbaubaren Polymerschicht mindestens ein Inhibitor des Transportproteins P-Glycoprotein sowie mindestens ein pharmazeutischer Wirkstoff befindet, **dadurch gekennzeichnet, dass** der mindestens ein Inhibitor des Transportproteins P-Glycoprotein ausgewählt ist aus der Gruppe bestehend aus PSC833, GF120918, XR9576, LY335979 und OC144-093.

2. Medizinprodukt nach Anspruch 1, **dadurch gekennzeichnet, dass** die biostabile und/oder bioabbaubare Polymerschicht zusammengesetzt ist aus Polymeren ausgewählt aus der Gruppe bestehend aus Polyolefine wie Polypropylen, Polyethylen, Polyisobutylen und Polybutylen, sowie Polyetherketone wie Polyetheretherketon, sowie Polyether wie Polyethylenglycol und Polypropylenglycol, sowie Polyvinylalkohole, Polyvinylhalogenide wie Polyvinylchlorid und Polyvinylfluorid, sowie Polyvinylester wie Polyvinylacetat, sowie Polyacrylate wie Polyethylacrylat, Polymethylacrylat und Polymethylmetacrylat, sowie Polyhalogenolefine wie Polytetrafluorethylen und Polychlortrifluorethylen, sowie Polyamide wie PA 11, PA 12, PA 46 und PA 66, sowie Polyamidimide, Polysulfone wie Polyethersulfon und Polyphenylsulfon, sowie Polyester wie Polycarbonate, Polybutylenterephthalat, Polyethylenterephthalat, sowie Polyurethane wie Elastane und Pellethane, sowie Silikone, Polyphosphazene, Polyphenylen, Polymerschäume (aus Styrolen und Carbonaten), Polydioxanone, Polyglycolide, Polylactide wie Poly-L-, Poly-D-, und Poly-D,L-Lactid, sowie Poly-εcaprolacton, Ethylvinylacetat, Polyethylenoxid, Polyphosphorylcholin, Polyhydroxybuttersäuren wie Polyhydroxyvalerat, sowie Lipide wie Cholesterin und Cholesterinester, sowie Polysaccharide wie Alginat, Chitosan, Levan, Hyaluronsäure, Uronide, Heparin, Dextran und Cellulose, sowie Proteine wie Fibrin und Albumin, sowie Polypeptide und Copolymere, Blends und Derivate dieser Verbindungen.

3. Medizinprodukt nach Anspruch 1 bis 2, **dadurch gekennzeichnet, dass** die auf der Oberfläche der Medizinprodukte aufgetragene biostabile und/oder bioabbaubare Polymerschicht eine Dicke von 2 µm bis 60 µm pro Schicht haben.

4. Medizinprodukt nach Anspruch 3, **dadurch gekennzeichnet, dass** die auf der Oberfläche der Medizinprodukte aufgetragene biostabile und/oder bioabbaubare Polymerschicht eine Dicke von 10 µm bis 30 µm pro Schicht haben.

5. Medizinprodukt nach einem der vorherigen Ansprüche, **dadurch gekennzeichnet, dass** sich in und/oder auf der biostabilen und/oder bioabbaubaren Polymerschicht der mindestens ein Inhibitor des Transportproteins P-Glycoprotein in einer Konzentration von 0,25 - 7,0 µg/mm² befindet.

6. Medizinprodukt nach einem der vorherigen Ansprüche, **dadurch gekennzeichnet, dass** der mindestens eine pharmazeutische Wirkstoff ein antimikrobieller, antimitotischer, antimyotischer, antineoplastischer, antiphlogistischer, antiproliferativer, antithrombischer und/oder vasodilatatorischer Wirkstoff ist ausgewählt aus der Gruppe bestehend aus Triclosan, Cephalosporin, Aminoglycosid, Nitrofurantoin, Penicilline wie Dicloxacillin, Oxacillin sowie Sulfonamide, Metronidazol, 5-Fluoruracil, Cisplatin, Vinblastin, Vincristin, Epothilonen, Endostatin, Verapamil, Statinen wie Cerivastatin, Atorvastatin, Simvastatin, Fluvastatin, Rosuvastatin sowie Lovastatin, Angiostatin, Angiopeptin, Taxanen wie Paclitaxel, Immunsuppressiva oder -modulatoren wie z.B. Rapamycin oder dessen Derivate wie Biolimus, Everolimus, Deforloimus, Novolimus, Methotrexat, Colchicin, Flavopiridol, Suramin, Cyclosporin A, Clotrimazol, Flucytosin, Griseofulvin, Ketoconazol, Miconazol, Nystatin, Terbinafin, Steroiden wie Dexamethason, Prednisolon, Corticosteron, Budesonid, Östrogen, Hydrocortison sowie Mesalamin, Sulfasalazin, Heparin und seinen Derivaten, Urokinase, PPack, Argatroban, Aspirin, Abciximab, synthetischem Antithrombin, Bivalirudin, Enoxoparin, Hirudin, r-Hirudin, Protamin, Prourokinase, Streptokinase, Warfarin, Flavonoiden wie 7,3',4'-trimethoxyflavon sowie Dipyramidol, Trapidil, Nitroprusside, einzeln oder in Kombination.

7. Medizinprodukt nach Anspruch 6, **dadurch gekennzeichnet, dass** der mindestens eine pharmazeutische Wirkstoff ausgewählt ist aus der Gruppe bestehend aus Paclitaxel, Rapamycin und dessen Derivate, Atorvastatin, Simvastatin, Lovastatin und Verapamil, einzeln oder in Kombinationen.

8. Medizinprodukt nach Anspruch 6, **dadurch gekennzeichnet, dass** der mindestens eine pharmazeutische Wirkstoff ausgewählt ist aus der Gruppe bestehend aus Paclitaxel und Rapamycin, einzeln oder in Kombination.

9. Medizinprodukt nach einem der vorherigen Ansprüche, **dadurch gekennzeichnet, dass** sich in und/oder auf der biostabilen und/oder bioabbaubaren Polymerschicht der mindestens eine pharmazeutische Wirkstoff in einer Konzentration von 0,2 - 3,5 µg/mm² befindet.

10. Medizinprodukt nach einem der vorherigen Ansprüchen, **dadurch gekennzeichnet, dass** das Medizinprodukt ein Ballonkatheter oder ein Stent ist.

11. Verwendung von PSC833 als Inhibitor des Transportproteins P-Glycoprotein zur Herstellung eines Medizinproduktes nach einem der Ansprüche 1 bis 10.

12. Verwendung von GF120918 als Inhibitor des Transportproteins P-Glycoprotein zur Herstellung eines Medizinproduktes nach einem der Ansprüche 1 bis 10.

13. Verwendung von XR9576 als Inhibitor des Transportproteins P-Glycoprotein zur Herstellung eines Medizinproduktes nach einem der Ansprüche 1 bis 10.

14. Verwendung von LY335979 als Inhibitor des Transportproteins P-Glycoprotein zur Herstellung eines Medizinproduktes nach einem der Ansprüche 1 bis 10.

15. Verwendung von OC144-093 als Inhibitor des Transportproteins P-Glycoprotein zur Herstellung eines Medizinproduktes nach einem der Ansprüche 1 bis 10.
